# EUROPEAN PATENT APPLICATION

(11) **EP 0 761 209 A2**
(43) Date of publication of application: **12.03.1997**
(21) Application number: 96100278.9
(22) Date of filing: 10.01.1996
(51) Int. Cl.: A61K 9/22, A61K 9/52, A61K 31/34

(54) **Controlled release formulations of ranitidine**

(30) Priority: 01.09.1995 US 522843
(71) Applicant: J.B. Chemicals & Pharmaceuticals Ltd., Worli, Bombay 400 025 (IN)
(72) Inventor: Mody, Shirish Bhagwanlal, Bombay 400 006 Maharashtra (IN); Doshi, Madhukant Mansukhlal Dr., Bombay 400 077, Maharashtra (IN); Joshi, Milind Dattatraya Dr., Thane 400 604, Maharashtra (IN)
(74) Representative: Weber, Dieter, Dr.

(57) **Abstract**

In oral formulations in form of coated tablets or capsules of the therapeutically active drug Ranitidine Hydrochloride, designed or intended to provide release of the active drug in a controlled manner over longer periods and containing pharmacologically acceptable dose of Ranitidine Hydrochloride blended with specified polymers, the quantity of the specified polymer is within the ratio of 0.2 : 1.2 with the quantity of the active drug, Ranitidine Hydrochloride.

## Description

The present invention relates to a pharmaceutical formulation designed to provide controlled release at regulated rates and within desired periods of the pharmacologically useful and active drug Ranitidine Hydrochloride, among others for anti-ulcer treatment.

Ranitidine Hydrochloride is a water soluble drug and readily dissolves and gets absorbed in the system soon after it is taken orally in form of tablet or capsule. Even when tablets are sugar coated or film coated in usual fashion, the dissolution and absorption takes places soon after the coating-film or sugar dissolves. As such the concentration of the drug in the system reaches peak as well as falls rapidly.Even with such increased dosages, the rate of release, and consequently the MEC levels cannot be maintained uniformly. For more effective treatment of gastric and intestinal ulcers, it is desirable and necessary to have a formulation which will maintain a level above minimum effective concentration and in a regulated manner (MEC) in the system for longer duration - preferably for 12 to 24 hours. The oral formulations of Ranitidine Hydrochloride presently produced and available are not designed to provide such controlled release. Consequently a regime of two or three doses per day is generally prescribed, and if the patient forgets or omits to take the second or third dose as prescribed, the efficacy of the treatment is considerably reduced.

Though this problem, and the need to provide a controlled release formulation of Ranitidine Hydrochloride, have been known since long, and the methods and substances generally employed to control rate of release, dissolution and absorption of different pharmaceutically useful and active drugs are also known, attempts to device such formulation for Ranitidine Hydrochloride administration, have not been successful so far and no such formulations were known. The solubility and the site, rate and period of release required and possibility of interaction with polymers presented special problem in case of Ranitidine Hydrochloride.

The present invention provides oral formulations in form of coated tablets and capsules which are designed for controlled or regulated dissolution and release at fairly uniform rate and over long periods - which may extend from 12 to 24 hours - to maintain at desired levels, the MEC of the pharmaceutically useful and active drug Ranitidine Hydrochloride. In vitro trials have shown that the drug is released at a controlled rate over long periods which can be extended upto 24 hours.

The new controlled release oral formulations of Ranitidine Hydrochloride comprising the new invention disclosed and claimed by this specification, can be prepared by the new process developed by the present inventors and more particularly described in the separate complete specification previously filed with the application No.16/BOM/95 at the Indian Patent office at Bombay on 11.01.1995 and is set out below for reference.
i) To a weighed quantity (taken proportionately to the dose of the active drug required in the final formulation) of Ranitidine Hydrochloride, are added required quantities of the specified polymer and the other necessary excipients and solvents, and the mass is thoroughly mixed and blended to make uniform paste, which is thereafter dried and granulated.
ii) For formulations in form of coated tablets, the granules so obtained are compressed into tablets of weight and size adjusted to give the required dose of the active drug adding lubricants or binding agents as required, and the tablets obtained are checked for in-process control tests such as dissolution rate, weight, appearance and the other relevant quality attributes, and the tested and approved tablets are thereafter given a coating, and the coating may also optionally contain a specified polymer. The coated tablets so obtained are subjected to quality control tests including in particular drug release test in compliance with the requirements.
iii) For formulations in form of capsules, the granules obtained as per No.(i) above, may optionally be given further coat with a coating solution containing a specified polymer depending on the rate and time of release desired. Required quantity of coated granules so obtained are then filled and encapsulated in capsules, and tested for quality standards and particularly to check the rate and time of dissolution of the active drug.

The excipients and solvents required to be used at various stages of the process are to be selected from out of those pharmaceutically acceptable and normally employed for the purpose. Those skilled in the art can decide as to the particular the excipients and solvents and quantities thereof to be employed in the process,having regard to the quantity of the active drug to be taken, and rates and periods of release of the active drug desired in the final formulation.

The expression "Specified Polymer' as used in this specification refers to any one or more of the pharmaceutically accepted inert polymer or mixture of such polymers normally employed and described in literature for coating of drugs for controlled release,out of which, the polymers - alkyl-celluloses such as methyl and ethyl-cellulose, polyvinyl- pyrrolidone, hydroxymethylcellulose, hydroxypropylcellulose,hydroxypropylmethyl cellulose, ( available under Trade Name Methocel K4M, K15M, K100M, or E, J, F grades) sodium carboxymethylcellulose, are the preferred polymers'. The other polymers which can also be used for the process, are polyethylene glycol, organic acids such as adipic acid, ascorbic acid,and such other polymers generally used, including copolymers (sold under the trade name "Eudragit") of varying permeability.

The quantity of the specified polymer to be taken for blending (stage (i) above) bears a ratio of 0.2 to 1.2 with the quantity of the active drug taken, the particular proportion and quantity of polymer to be decided by those skilled in the art, having regard to the rate and time of release of the active drug required in the final formulation. The best results are obtained using the preferred polymers' mentioned above.

The solvents to be used for the process are pharmaceutically acceptable solvents, particular solvent being selected on basis of the solubility of the polymer and drug employed.

The solvent is suitably selected from water, alcohols, ketones, halogenated aliphatic compounds, halogenateed aromatic hydrocarbon compounds, aromatic hydrocarbon compounds and cyclic ethers or a mixture thereof, specially preferred solvents to be used in the process include water, hexane, heptane, methanol, ethanol, isopropyl alcohol, acetone, methylethyl ketone, methylisobutyl ketone, methylene chloride, chloroform, carbon tetrachloride, toluene, xylene and tetrahydrofuran.

The process as described in this specification yields the tablet/capsule formulations with controlled rate of delivery of the active drug.

The invention will be further illustrated with reference to the following examples, but do not limit the scope thereof.

### EXAMPLE 1

Ranitidine Hydrochloride 336.0 g equivalent to Ranitidine 300 g is mixed with the following ingredients :-
Hydroxypropylmethyl cellulose K 15 M - 77.0 g.
Hydroxypropylmethyl Cellulose K 100 M -35.0 g.
in presence of solvents Isopropyl alcohol (500 ml) and methylene chloride (300 ml.)

The granules were dried and then tabletted with the aid of Magnesium Stearate (2 g) as a lubricant, to obtain a tablet equivalent to 300 mg of active ingredient, and having controlled rate of dissolution and drug release as desired.

The compressed tablets were further coated with coating solution consisting of

| | |
|---|---|
| Hydroxypropylmethyl cellulose | 2.0 g |
| Polyethylene glycol | 1.0 g |
| Titanium Dioxide | 0.2 g |
| Methylene chloride (25 ml) Isopropyl alcohol (25 ml) | |

In the above Example, the ratio of Ranitidine Hydrochloride, Hydroxypropylmethyl cellulose K 15 M and Hydroxypropylmethyl cellulose K 100 M may also be suitably altered within the limits, which while retaining the characteristic nature of the drug, may provide release of the effective drug at a different rate and period as may be desired. This also applies in the case of the subsequent examples.

### EXAMPLE 2

Ranitidine Hydrochloride 168.0 g equivalent to Ranitidine 150 g is mixed with the following ingredients :-
Hydroxypropylmethyl cellulose K 15 M - 50.0 g.
Hydroxypropylmethyl cellulose K 100 M - 62.0 g.
in presence of solvents Isopropyl alcohol (500 ml) and methylene chloride (300 ml.)

The granules were dried and then tabletted with the aid of Magnesium Stearate (2 g) as a lubricant, to obtain a tablet containing equivalent to 150 mg of active ingredient, and having rate of dissolution & drug release as desired.

The compressed tablets were further coated with coating solution consisting of

| | |
|---|---|
| Hydroxypropylmethyl cellulose | 2.0 g |
| Polyethylene glycol | 1.0 g |
| Titanium Dioxide | 0.2 g |
| Methylene chloride (25 ml) Isopropyl alcohol (25 ml) | |

### EXAMPLE 3

Ranitidine Hydrochloride 336.0 g equivalent to Ranitidine 300 g is mixed and granulated with the following ingredients :-
Hydroxypropylmethyl cellulose K 15 M - 75.0 g.
Hydroxypropylmethyl cellulose K 100 M - 37.0 g.
in presence of solvents Isopropyl alcohol (500 ml) and methylene chloride (300 ml.)

The granules so obtained were further coated with coating solution consisting of:

| | |
|---|---|
| Hydroxypropylmethyl cellulose | 2.0 g |
| Polyethylene glycol | 1.0 g |
| Titanium Dioxide | 0.2 g |
| Methylene chloride (25 ml) Isopropyl alcohol (25 ml) | |

The coated granules were then encapsulated in a conventional manner to obtain capsules containing 300 mg of active ingredient, and having controlled rate of dissolution & drug release as desired.

### EXAMPLE 4

The procedure employed was similar to that in Example 1 except that the amount of Hydroxypropylmethyl cellulose K-15 M taken was 75 g and also included was carboxymethyl cellulose sodium (5 g).

### EXAMPLE 5

The procedure employed was similar to that in Example 1 except that the Hydroxypropylmethyl cellulose K 15 M was replaced by methyl methacrylates (75 g).

### EXAMPLE 6

The procedure employed was similar to that in Example 1 except that polyvinylpyrrolidone was used instead of Hydroxypropyl methylcellulose,K 15 M.

The formulations obtained on following the procedure as per each of the above examples, when tested in vitro show release of the active drug in a controlled manner for maintaining MEC levels over longer duration ranging from 12 hours to 24 hours.

These processes are only examples (embodiments of the invention) and there would be other process variation for production of such products. Such processes are also within the scope of this invention.

## Claims

1. Oral formulations in form of coated tablets or capsules of the therapeutically active drug Ranitidine Hydrochloride, designed or intended to provide release of the active drug in a controlled manner over longer periods and containing pharmacologically acceptable dose of Rantidine Hydrochloride blended with specified polymers, the quantity of the specified polymer being within the ratio of 0.2 to 1.2 with the quantity of the active drug, Rantidine Hydrochloride, and such oral formulations answering the description - in case of coated tablets, as set out in claim 2 below, and in case of capsules, as set out in claim 3 below.

2. An oral formulation for controlled release and delivery of the drug Ranitidine Hydrochloride in the form of coated tablets containing Ranitidine Hydrochloride in pharmacologically acceptable doses blended with an inert polymer and other excipients as required, and such tablets may optionally have a further coating also containing a polymer, wherein the particular polymer and excipients and quantity thereof are determined and added according to the rate and period of release of the active drug desired.

3. An oral formulation for controlled release and delivery of the drug Ranitidine Hydrochloride in the form of capsules containing granules of Ranitidine Hydrochloride in pharmacologically acceptable dose blended with an inert polymer and other excipients as required, and such granules may optionally have a further coating also containing a polymer, wherein the particular polymer and excipients and quantity thereof are determined and added according to the rate and period of release of the active drug desired.

4. An oral formulation for controlled release and delivery of the drug Ranitidine Hydrochloride in the form of coated tablets substantially as claimed in claim No.2 above with the coating being film coating which may optionally contain polymer as mentioned therein.

5. An oral formulation for controlled release and delivery of the drug Ranitidine Hydrochloride in the form of coated tablets substantially as claimed in claim No.2 above, but with a sugar coating which may optionally contain polymer as mentioned therein.

6. An oral formulation for controlled release and delivery of the drug Ranitidine Hydrochloride in the form of coated tablets substantially as described in claim 2 above, wherein the quantity of Ranitidine Hydrochloride taken is equivalent to 150 mg or 300 mg or other pharmacologically acceptable dose of active drug.

7. An oral formulation for controlled release and delivery of the drug Ranitidine Hydrochloride in the form of capsules substantially as described in claim No.3 above, wherein Ranitidine Hydrochloride taken is equivalent to 150 mg or 300 mg or other pharmacologically acceptable dose of active drug.

8. An oral formulation for controlled release and delivery of the drug Ranitidine Hydrochloride in the form of coated tablets or capsules (as the case may be) as per any one of the claims 1-7 above wherein instead of one polymer, a mixture of polymers out of the preferred variety is used.

9. An oral formulation for controlled release and delivery of the drug Ranitidine Hydrochloride in the form of coated tablets prepared as mentioned in Example 1 above.

10. An oral formulation for controlled release and delivery of the drug Ranitidine Hydrochloride in the form of coated tablets prepared as mentioned in Example 2 above.

11. An oral formulation for controlled release and delivery of the drug Ranitidine Hydrochloride in the form of capsules prepared as mentioned in Example 3 above.

12. An oral formulation for controlled release and delivery of the drug Ranitidine Hydrochloride in the form of coated tablets prepared as mentioned in Example 4 above.

13. An oral formulation for controlled release and delivery of the drug Ranitidine Hydrochloride in the form of coated tablets prepared as mentioned in Example 5 above.

14. An oral formulation for controlled release and delivery of the drug Ranitidine Hydrochloride in the form of coated tablets prepared as mentioned in Example 6 above.
